# EUROPEAN PATENT APPLICATION

(11) **EP 3 170 467 A1**
(43) Date of publication of application: **24.05.2017**
(21) Application number: 15822038.4
(22) Date of filing: 22.06.2015
(51) Int. Cl.: A61B 18/00

(54) **ULTRASONIC VIBRATOR FOR MEDICAL TREATMENT**

(30) Priority: 18.07.2014 JP 2014147803
(71) Applicant: OLYMPUS CORPORATION, Hachioji-shi Tokyo 192-8507 (JP)
(72) Inventor: TODA, Masaya, Tokyo 192-8507 (JP)
(74) Representative: Schicker, Silvia
(86) International application number: PCT/JP2015/067901
(87) International publication number: WO 2016/009788

(57) **Abstract**

A therapeutic ultrasonic transducer (10) according to the present invention is provided with: a transducer body (1) that has, in the stated order along the longitudinal axis, a first metal body (4), a laminated body (5) in which a plurality of piezoelectric elements are laminated in the longitudinal-axis direction such that their directions of polarization are set in opposite directions in an alternating manner, and a second metal body (6), and that is formed by integrally fastening the metal bodies (4 and 6) and the laminated body (5) with a bolt (7); and a heat-radiation means (2) that is connected at a position, on the transducer body (1), serving as a node of an ultrasonic vibration, or in the vicinity of that position. The therapeutic ultrasonic transducer (10) produces an ultrasonic vibration having a frequency of 20 kHz or higher and 100 kHz or lower.

## Description

### {Technical Field}

The present invention relates to a therapeutic ultrasonic transducer and particularly to a bolt-clamped Langevin therapeutic ultrasonic transducer to be mounted in an ultrasonic treatment tool.

### {Background Art}

A bolt-clamped Langevin transducer (BLT) is conventionally used as an ultrasonic transducer for an ultrasonic treatment tool (for example, see PTLs 1 and 2). The BLT transducer is obtained by sandwiching a piezoelectric element between a pair of high-stiffness metal bodies made of aluminum alloy, titanium alloy, or the like and by tightly fastening the piezoelectric element and the pair of metal bodies with a bolt. This transducer can produce powerful ultrasonic vibrations.

The treatment performance of an ultrasonic treatment tool depends on the strength of ultrasonic vibrations produced by the ultrasonic transducer. However, when the electric power supplied to the ultrasonic transducer is increased in order to enhance the ultrasonic vibrations, the amount of heat generated in the ultrasonic transducer increases. Thus, there is a known ultrasonic treatment tool that is provided with a means for promoting heat radiation from the ultrasonic transducer (for example, see PTL 3). In the ultrasonic treatment tool of PTL 3, heat-radiation fins are provided on the outer surface of a case for containing the ultrasonic transducer, thereby achieving the release of heat transferred from the ultrasonic transducer to the case.

### {Citation List}

### {Patent Literature}

{PTL 1} Publication of Japanese Patent No. 4642935
{PTL 2} Japanese Unexamined Patent Application, Publication No. Sho 61-18299
{PTL 3} Japanese Unexamined Patent Application, Publication No. 2001-321388

### {Summary of Invention}

### {Technical Problem}

In a case in which hard tissue, such as bone, cartilage, or calcified tissue, undergoes treatment, especially-powerful ultrasonic vibrations are required, and the electric power supplied to the ultrasonic transducer needs to be increased. With such a use, the amount of heat generated in the ultrasonic transducer exceeds the heat-radiation performance of the heat-radiation fins of PTL 3, and thus, there is a problem in that the heat-radiation fins of PTL 3 cannot suppress overheating of the ultrasonic transducer. There is a disadvantage that, once overheating occurs in the ultrasonic transducer, the treatment has to be temporarily stopped until the temperature of the ultrasonic transducer decreases to fall within a normal range, thus lengthening the entire treatment time.

The present invention has been made in view of the above-described circumstances, and an object thereof is to provide a therapeutic ultrasonic transducer capable of effectively suppressing overheating and continuously exhibiting high treatment performance.

### {Solution to Problem}

In order to achieve the above-described object, the present invention provides the following solutions.

The present invention provides a therapeutic ultrasonic transducer that produces an ultrasonic vibration having a frequency of 20 kHz or higher and 100 kHz or lower, including: a transducer body that has, in order from a distal end along a longitudinal axis, a first metal body, a laminated body in which a plurality of piezoelectric elements are laminated in the longitudinal-axis direction such that their directions of polarization are set in opposite directions in an alternating manner, and a second metal body, and that is formed by integrally fastening the first metal body, the laminated body, and the second metal body with a bolt; and a heat-radiation means that is connected at a position, on the transducer body, serving as a node of the ultrasonic vibration, or in the vicinity of that position and that releases heat in the transducer body to the outside of the transducer body.

According to the present invention, ultrasonic vibrations produced by the transducer body when high-frequency power is supplied to the piezoelectric elements are applied to tissue directly from the transducer body or via another member, thereby making it possible to apply treatments, such as pulverization and dissolution, to the tissue.

In this case, heat in the transducer body is released with high efficiency by the heat-radiation means, which is connected to the transducer body at the position of a node at which the amount of distortion due to the ultrasonic vibrations becomes maximum, and the amount of heat generation becomes maximum; thus, overheating of the transducer body is effectively suppressed. Therefore, when high-frequency power is supplied to the piezoelectric elements, the transducer body can continuously exhibit high treatment performance without causing overheating.

In the above-described invention, it is preferred that the transducer body have, at a distal end of the first metal body, a horn that is integrally connected to the first metal body, and that the connection position in the longitudinal-axis direction between the first metal body and the horn be a position at which the ratio of the vibration velocity to the vibration velocity at the position of an antinode of the ultrasonic vibration becomes 0.05 or greater and 0.3 or less.

By doing so, while the boundary plane between the relatively-fragile horn and the first metal body is arranged at a position shifted from the position of the node, at which a large stress is produced, it is possible to obtain an excellent heat-radiation effect from the node, which is located at an intermediate position of the horn in the longitudinal-axis direction.

In the above-described invention, the heat-radiation means may be provided with a cylindrical heat-radiation tube that accommodates the transducer body in the longitudinal-axis direction and that has, on the outer periphery thereof, a plurality of heat-radiation fins projecting radially outward.

By doing so, with the large-area heat-radiation tube, which covers the outer periphery of the transducer body, it is possible to obtain high heat-radiation performance, practically without increasing the entire diameter size.

In the above-described invention, the transducer body may be provided with, at the position serving as the node or in the vicinity thereof, a flange portion projecting radially outward; and the heat-radiation means may be provided with a Peltier element fixed to the flange portion.

By doing so, the Peltier element actively absorbs heat from the transducer body, thereby making it possible to obtain high heat-radiation performance.

In the above-described invention, the first metal body and the second metal body may have a plurality of heat-radiation grooves formed on the outer peripheries thereof.

In the above-described invention, the second metal body may be provided with, on a base-end surface thereof, a plurality of heat-radiation fins that extend in the longitudinal-axis direction.

By doing so, heat radiation is effectively performed not only from the position of the node but also from the outer surfaces of the metal bodies, and thus, the heat-radiation performance can be further improved.

### {Advantageous Effects of Invention}

According to the present invention, an advantageous effect is afforded in that it is possible to effectively suppress overheating and to continuously exhibit high treatment performance.

### {Brief Description of Drawings}

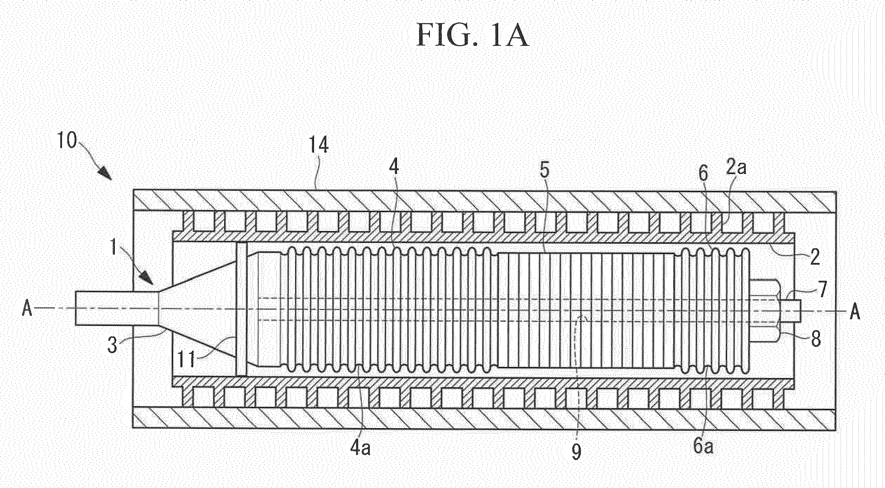

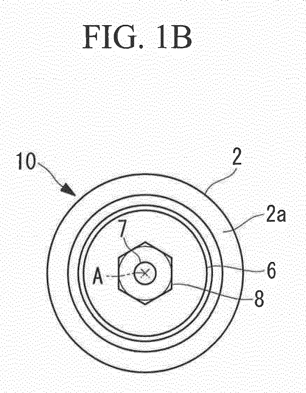

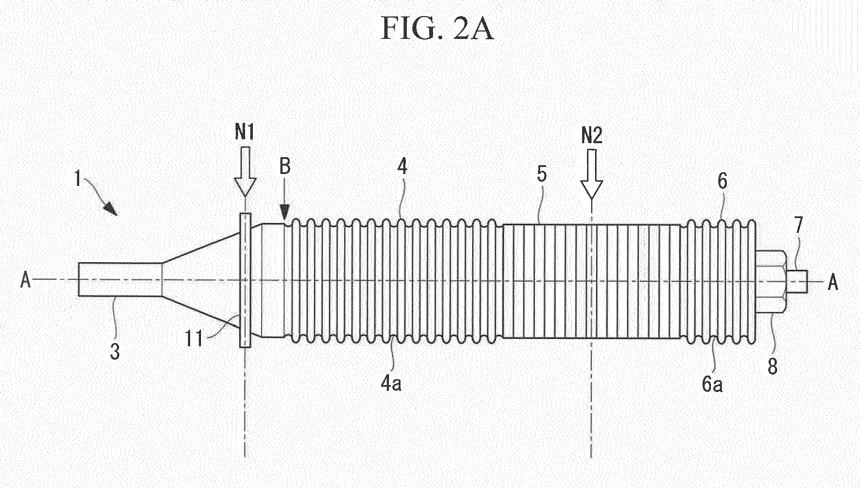

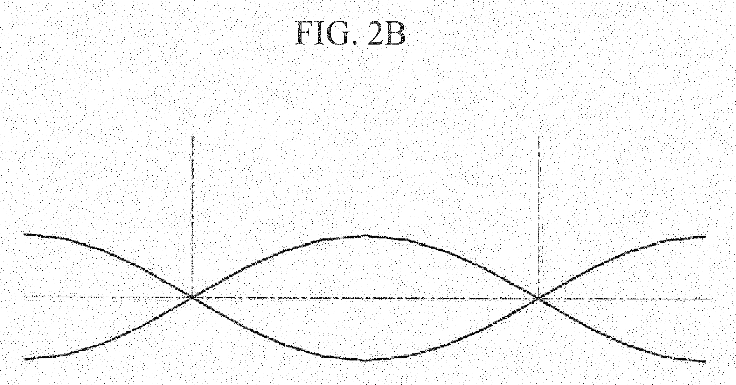

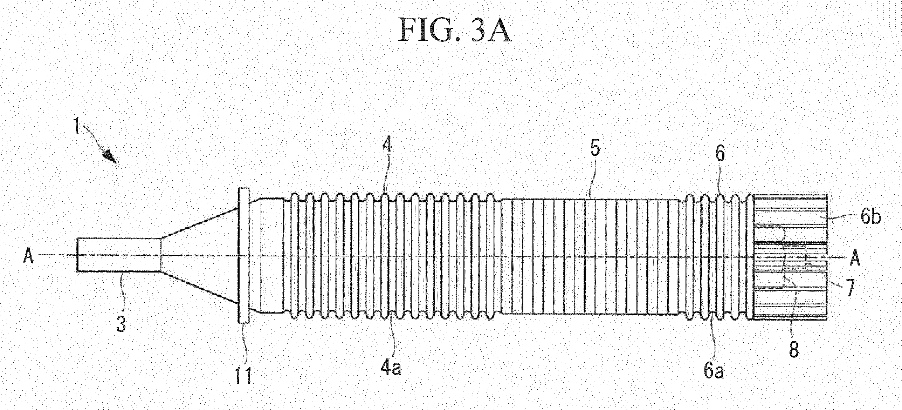

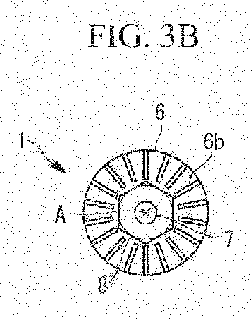

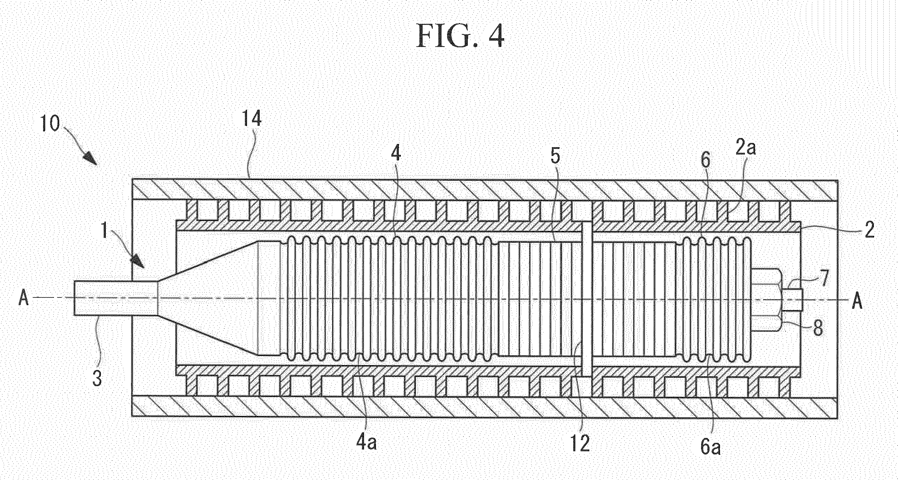

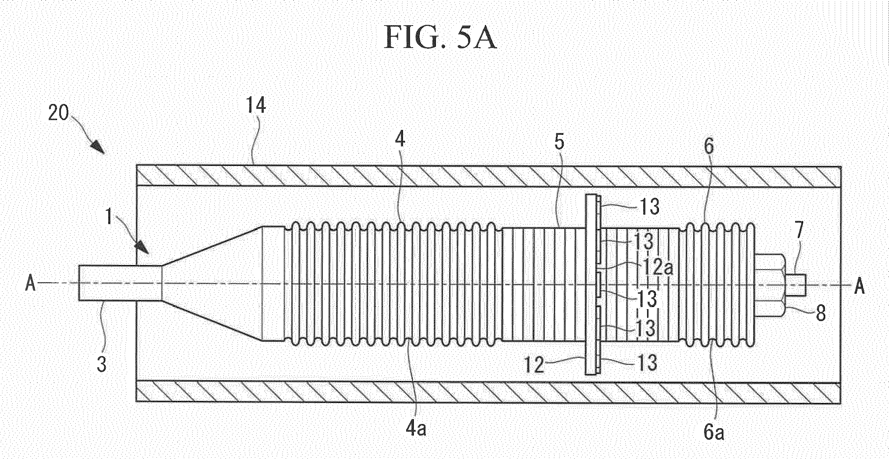

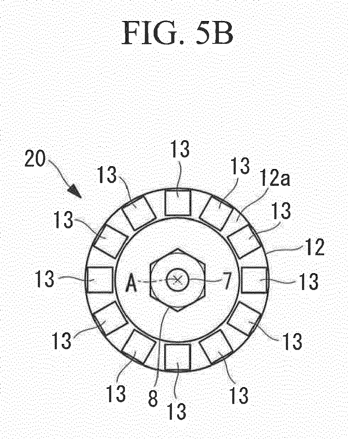

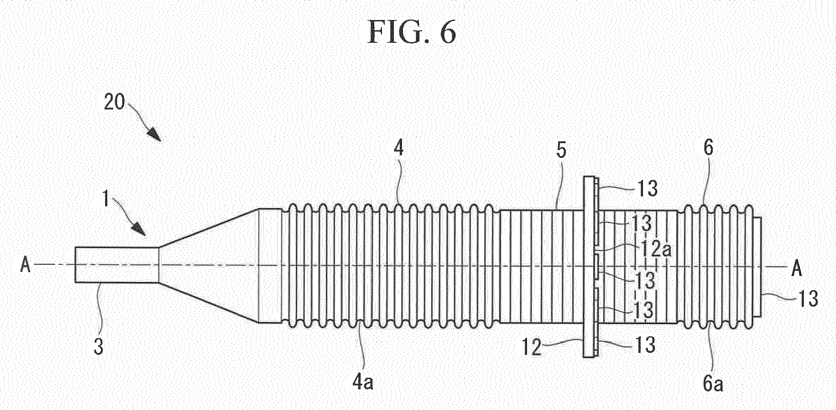

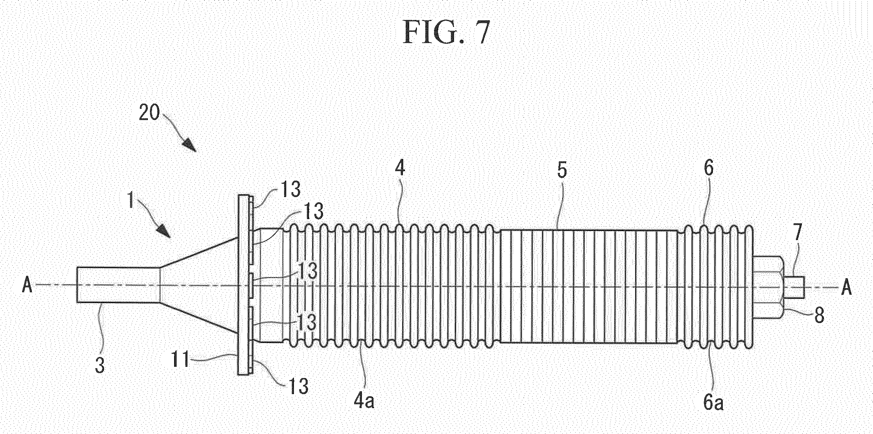

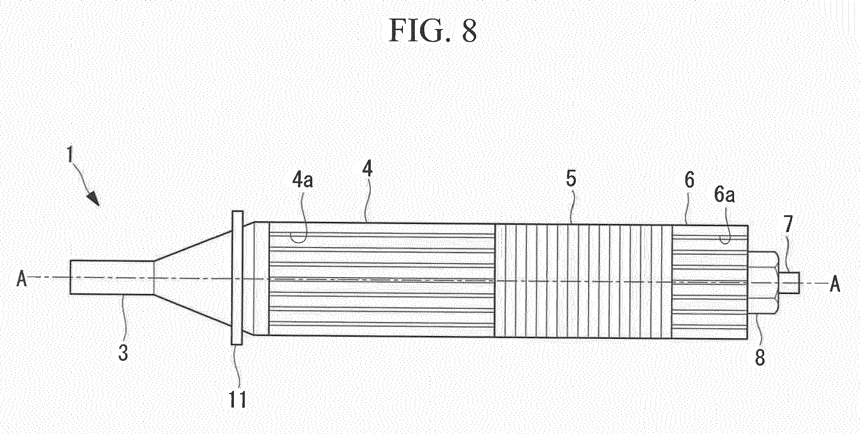

{Fig. 1A} Fig. 1A is a side view showing the overall configuration of an ultrasonic transducer according to a first embodiment of the present invention.
{Fig. 1B} Fig. 1B is a rear view of the ultrasonic transducer shown in Fig. 1A, viewed in a longitudinal-axis direction from a base end.
{Fig. 2A} Fig. 2A is a view showing the positions of nodes in a longitudinal vibration of the ultrasonic transducer shown in Fig. 1A.
{Fig. 2B} Fig. 2B is a view showing displacement in the longitudinal vibration at each position on the ultrasonic transducer shown in Fig. 1A in the longitudinal-axis direction.
{Fig. 3A} Fig. 3A is a side view showing a modification of the ultrasonic transducer shown in Fig. 1A.
{Fig. 3B} Fig. 3B is a rear view of an ultrasonic transducer shown in Fig. 3A, viewed in the longitudinal-axis direction from the base end.
{Fig. 4} Fig. 4 is a side view showing another modification of the ultrasonic transducer shown in Fig. 1A.
{Fig. 5A} Fig. 5A is a side view showing the overall configuration of an ultrasonic transducer according to a second embodiment of the present invention.
{Fig. 5B} Fig. 5B is a rear view of the ultrasonic transducer shown in Fig. 5A, viewed in the longitudinal-axis direction from the base end.
{Fig. 6} Fig. 6 is a side view showing a modification of the ultrasonic transducer shown in Fig. 5A.
{Fig. 7} Fig. 7 is a side view showing another modification of the ultrasonic transducer shown in Fig. 5A.
{Fig. 8} Fig. 8 is a side view showing a modification of transducer bodies shown in Fig. 1A and Fig. 5A.

### {Description of Embodiments}

### First Embodiment

A therapeutic ultrasonic transducer 10 according to a first embodiment of the present invention will be described with reference to Fig. 1A to Fig. 4.

As shown in Figs. 1A and 1B, the therapeutic ultrasonic transducer 10 of this embodiment is provided with a transducer body (hereinafter, simply referred to as "body") 1 that is formed of a bolt-clamped Langevin transducer (BLT) and a heat-radiation tube 2 that accommodates the body 1. Reference sign 14 denotes an outer cylinder having electrical insulation properties, for covering the outer side of the therapeutic ultrasonic transducer 10.

The body 1 is provided with, in order from a distal end along a longitudinal axis A, a horn 3, a first metal body 4, a laminated body 5 composed of a plurality of piezoelectric elements, and a second metal body 6. Furthermore, the body 1 is provided with a bolt 7 and a nut 8 that are used to integrally fasten the first metal body 4, the laminated body 5, and the second metal body 6.

The piezoelectric elements are plate-like members made of a piezoelectric material, such as PZT (lead zirconate titanate), and polarize in the thickness direction. In the laminated body 5, the plurality of piezoelectric elements are laminated in the direction of the longitudinal axis A such that their directions of polarization are set in opposite directions in an alternating manner.

The first metal body 4 and the second metal body 6 are columnar members made of alloy consisting primarily of aluminum or made of a ceramic (for example, duralumin). A number of heat-radiation grooves 4a and 6a that extend in the circumferential direction are formed on the outer peripheries of the metal bodies 4 and 6 at intervals in the direction of the longitudinal axis A. With the heat-radiation grooves 4a and 6a, the surface areas of the metal bodies 4 and 6 are increased, thus promoting heat radiation from the metal bodies 4 and 6.

The horn 3 and the bolt 7 are individual members made of metal having a high ultrasonic-propagation efficiency and a high strength. The horn 3 and the bolt 7 are preferably made of 64 titanium alloy (ASTM B348 Grade5). The horn 3 has a substantially conical shape tapering toward the distal end. The bolt 7 linearly extends along the longitudinal axis A from a base-end surface of the horn 3 toward a base end.

The first metal body 4, the laminated body 5, and the second metal body 6 have a bolt hole 9 that is formed to penetrate therethrough along the longitudinal axis A and into which the bolt 7 is inserted. The nut 8 is fastened to a distal end portion of the bolt 7, which protrudes from a base-end surface of the second metal body 6, and thus, the laminated body 5 is tightly fastened by the first metal body 4 and the second metal body 6 from both sides. Note that the nut 8 may be omitted, and the second metal body 6 may have an internal thread to be fastened to the bolt 7, thus serving as the nut 8.

When high-frequency power from a high-frequency power source (not shown) is applied to the laminated body 5, the laminated body 5 produces a longitudinal vibration in the direction of the longitudinal axis A, the produced longitudinal vibration is transferred to the horn 3 via the bolt 7, and the distal end of the horn 3 is vibrated in the direction of the longitudinal axis A. At this time, the longitudinal vibration is amplified while being transferred from the base end of the horn 3 to the distal end thereof, thereby obtaining a large-amplitude vibration at the distal end of the horn 3. Here, the frequency of the high-frequency power is selected from the range from 20 kHz to 100 kHz such that the distal end of the body 1, an intermediate position thereof, and the base end thereof serve as antinodes of the longitudinal vibration, as shown in Figs. 2A and 2B. In Fig. 2A, arrows N1 and N2 indicate nodes of the longitudinal vibration.

The body 1 is further provided with, at the position of the node N1, which is close to the distal end, or in the vicinity thereof, a ring-like flange portion 11 that projects radially outward and that is made of metal having high thermal conductivity. Specifically, the position of the flange portion 11 attached to the body 1 in the direction of the longitudinal axis A is a position at which the vibration velocity becomes zero.

When the vibration velocity of the longitudinal vibration in a boundary plane B between the metal body 4 and the horn 3, which has the flange portion 11, is V1, and the vibration velocity of the longitudinal vibration at the position of an antinode is V2, it is preferable that the vibration velocity ratio V1/V2 be 0.05 or larger and 0.3 or smaller. When the vibration velocity ratio V1/V2 is larger than 0.3, the horn 3 is connected to the metal body 4 at a position where the vibration velocity is high, thereby producing a vibration, such as a shifted vibration, other than the original longitudinal vibration in the direction of the longitudinal axis A, and making it easy to generate heat, and there is a possibility that the heat-radiation performance of the heat-radiation tube 2, to be described later, cannot be sufficiently obtained. On the other hand, if the boundary plane B between different types of metals is arranged at the node N1, this can lead to strength poverty. Thus, it is preferred that the boundary plane B between the horn 3 and the metal body 4 be arranged at a position that is slightly distant from the node N1 and at which the vibration velocity ratio V1/V2 becomes 0.05 or larger.

The heat-radiation tube 2 is a cylindrical member made of metal having high thermal conductivity and accommodates the body 1, with a distal end portion of the horn 3 protruding from an opening at the distal end of the heat-radiation tube 2. A number of heat-radiation fins 2a that extend in the circumferential direction are provided on the outer periphery of the heat-radiation tube 2 so as to be arranged at intervals in the direction of the longitudinal axis A. The heat-radiation tube 2 is fixed to the outer periphery of the flange portion 11.

Next, the operation of the thus-configured therapeutic ultrasonic transducer 10 will be described.

The therapeutic ultrasonic transducer 10 of this embodiment is mounted in an ultrasonic treatment apparatus that is used to apply ultrasonic vibrations to tissue in a living body, thereby applying treatments, such as pulverization and dissolution, to the tissue. When high-frequency power having a frequency that falls within the range from 20 kHz to 100 kHz is supplied to the laminated body 5 from a high-frequency power source provided in the ultrasonic treatment apparatus, the laminated body 5 produces a longitudinal vibration in the direction of the longitudinal axis A, and the distal end of the horn 3 performs ultrasonic vibrations. Therefore, the distal end of the horn 3, which performs the ultrasonic vibrations, is brought into contact with an affected area, thereby making it possible to apply treatments, such as pulverization and dissolution, to the affected area.

In this case, according to this embodiment, although most of the high-frequency power supplied to the laminated body 5 is converted into mechanical vibrations, part thereof is converted into heat. The heat generated in the body 1 is released to the outside of the heat-radiation tube 2 by the heat-radiation grooves 4a and 6a, which are provided on the metal bodies 4 and 6, and the heat-radiation tube 2, which is provided at an outer side of the body 1, and is further released to the outside of the outer cylinder 14. Accordingly, overheating of the body 1 is suppressed. In particular, the amount of heat generation becomes maximum at the positions of the nodes N1 and N2 on the body 1, where the amount of distortion becomes maximum. Because the heat-radiation tube 2 is coupled at the position of the node N1 on the body 1 via the flange portion 11, the heat in the body 1 is released via the heat-radiation tube 2 with high efficiency. Therefore, even when the high-frequency power supplied to the laminated body 5 is increased in order to enhance the treatment performance by increasing the vibration amplitude of the distal end of the horn 3, there is an advantage that the therapeutic ultrasonic transducer 10 can be continuously operated without overheating and can continuously exhibit high treatment performance.

Furthermore, conventional BLT transducers generally use metal bodies made of titanium; however, the therapeutic ultrasonic transducer 10 of this embodiment uses the metal bodies 4 and 6, which are made of aluminum having much higher thermal conductivity than titanium. Therefore, there is an advantage that heat generated in the laminated body 5 can be more effectively released via the metal body 4 and the heat-radiation tube 2, and the entire length of the body 1 can be shortened.

In this embodiment, as shown in Figs. 3A and 3B, a plurality of heat-radiation fins 6b that extend in the direction of the longitudinal axis A may be provided at intervals on a base-end surface of the second metal body 6. In the example shown in Figs. 3A and 3B, the heat-radiation fins 6b are provided so as to be arranged in the circumferential direction, with the longitudinal axis A located at the center.

In the body 1, only the distal end portion of the horn 3 performs treatment on tissue. Thus, the heat-radiation fins 6b are provided at the base end of the body 1, thereby making it possible to further improve the heat-radiation performance of the therapeutic ultrasonic transducer 10 without affecting the treatment action of the therapeutic ultrasonic transducer 10.

In this embodiment, the body 1 and the heat-radiation tube 2 are connected via the flange portion 11 at the node N1, which is close to the distal end, of the two nodes N1 and N2 of the longitudinal vibration; however, instead of this, or, in addition to this, the body 1 and the heat-radiation tube 2 may be connected at the node N2, which is close to the base end, as shown in Fig. 4.

In this case, the node N2 is located at the laminated body 5. Therefore, a metal plate 12 sandwiched between two piezoelectric elements is further provided. The metal plate 12 has a larger diameter than the piezoelectric elements, a peripheral portion of the metal plate 12 radially projects in a flanged manner from the outer periphery of the laminated body 5. The heat-radiation tube 2 is coupled and fixed to the peripheral portion or the outer periphery of the metal plate 12.

With this configuration, it is possible to obtain the same heat-radiation effect as that obtained with the configuration shown in Figs. 1A and 1B.

### Second Embodiment

Next, a therapeutic ultrasonic transducer 20 according to a second embodiment of the present invention will be described with reference to Fig. 5A to Fig. 7.

The therapeutic ultrasonic transducer 20 of this embodiment differs from the therapeutic ultrasonic transducer 10 of the first embodiment in that Peltier elements 13 are provided instead of the heat-radiation tube 2. Therefore, in this embodiment, a description will be given mainly of the Peltier elements 13, identical reference signs are assigned to the other configurations common to those of the first embodiment, and a description thereof will be omitted.

As shown in Figs. 5A and 5B, the therapeutic ultrasonic transducer 20 is provided with a metal plate 12. The metal plate 12 is equivalent to the metal plate 12 shown in Fig. 4. The plurality of Peltier elements 13 are fixed, with an adhesive, to a ring-like peripheral portion (flange portion) 12a of the metal plate 12, which radially projects in a flanged manner from the outer periphery of the laminated body 5, so as to be arranged in the circumferential direction such that endothermic surfaces thereof are in contact with the surface of the metal plate 12. When an electric current is supplied through an electric wire (not shown), each of the Peltier elements 13 absorbs heat from the laminated body 5 via the metal plate 12 and releases the heat from a heat-radiation surface opposing the endothermic surface.

According to the thus-configured therapeutic ultrasonic transducer 20, heat generated in the body 1 by supplying high-frequency power to the laminated body 5 is absorbed by the Peltier elements 13, is released from the heat-radiation surfaces of the Peltier elements 13, and is further released to the outside of the outer cylinder 14. Accordingly, overheating of the body 1 is suppressed. In particular, the Peltier elements 13 are connected at the position of the node N2 on the body 1, where the amount of heat generation becomes maximum, via the metal plate 12; therefore, heat in the body 1 is released by the Peltier elements 13 with high efficiency. Therefore, even when the high-frequency power supplied to the laminated body 5 is increased in order to enhance the treatment performance by increasing the vibration amplitude of the distal end of the horn 3, there is an advantage that the therapeutic ultrasonic transducer 20 can be continuously operated without overheating and can continuously exhibit high treatment performance. The other advantageous effects are the same as those in the first embodiment, and thus, a description thereof will be omitted.

In this embodiment, as shown in Fig. 6, a Peltier element 13 may be provided also at the base-end surface of the second metal body 6. Fig. 6 shows a second metal body 6 that also serves as a nut, and the Peltier element 13 is fixed to the flat base-end surface of the second metal body 6. By doing so, it is possible to further improve the heat-radiation performance of the therapeutic ultrasonic transducer 20 without affecting the treatment action of the therapeutic ultrasonic transducer 20.

In this embodiment, the Peltier elements 13 are connected at the position of the node N2, which is close to the base end of the body 1, via the metal plate 12; however, instead of this, or, in addition to this, as shown in Fig. 7, the Peltier elements 13 may be connected at the position of the node N1, which is close to the distal end of the body 1. In this case, the Peltier elements 13 need to be fixed to the surface of the above-described flange portion 11.

Furthermore, the heat-radiation tube 2, which is described in the first embodiment, and the Peltier elements 13, which are described in the second embodiment, may be appropriately combined and used.

In the first and second embodiments, the shapes of the heat-radiation grooves 4a and 6a and the heat-radiation fins 2a can be appropriately changed. For example, as shown in Fig. 8, the heat-radiation grooves 4a and 6a may be formed in the direction of the longitudinal axis A at intervals in the circumferential direction. Similarly, the heat-radiation fins 2a may be formed in the direction of the longitudinal axis A at intervals in the circumferential direction.

### {Reference Signs List}

- 1: transducer body
- 2: heat-radiation tube (heat-radiation means)
- 2a: heat-radiation fins
- 3: horn
- 4, 6: metal body
- 4a, 6a: heat-radiation grooves
- 5: laminated body
- 6b: heat-radiation fins
- 7: bolt
- 8: nut
- 9: bolt hole
- 10, 20: therapeutic ultrasonic transducer
- 11: flange portion
- 12: metal plate
- 12a: peripheral portion (flange portion)
- 13: Peltier element (heat-radiation means)

## Claims

1. A therapeutic ultrasonic transducer that produces an ultrasonic vibration having a frequency of 20 kHz or higher and 100 kHz or lower, comprising:
a transducer body that has, in order from a distal end along a longitudinal axis, a first metal body, a laminated body in which a plurality of piezoelectric elements are laminated in the longitudinal-axis direction such that their directions of polarization are set in opposite directions in an alternating manner, and a second metal body, and that is formed by integrally fastening the first metal body, the laminated body, and the second metal body with a bolt; and
a heat-radiation means that is connected at a position, on the transducer body, serving as a node of the ultrasonic vibration, or in the vicinity of that position and that releases heat in the transducer body to the outside of the transducer body.

2. A therapeutic ultrasonic transducer according to claim 1,
wherein the transducer body has, at a distal end of the first metal body, a horn that is integrally connected to the first metal body; and
the connection position in the longitudinal-axis direction between the first metal body and the horn is a position at which the ratio of the vibration velocity to the vibration velocity at the position of an antinode of the ultrasonic vibration becomes 0.05 or greater and 0.3 or less.

3. A therapeutic ultrasonic transducer according to claim 1 or 2, wherein the heat-radiation means is provided with a cylindrical heat-radiation tube that accommodates the transducer body in the longitudinal-axis direction and that has, on the outer periphery thereof, a plurality of heat-radiation fins projecting radially outward.

4. A therapeutic ultrasonic transducer according to one of claims 1 to 3,
wherein the transducer body is provided with, at the position serving as the node or in the vicinity thereof, a flange portion projecting radially outward; and
the heat-radiation means is provided with a Peltier element fixed to the flange portion.

5. A therapeutic ultrasonic transducer according to one of claims 1 to 4, wherein the first metal body and the second metal body have a plurality of heat-radiation grooves formed on the outer peripheries thereof.

6. A therapeutic ultrasonic transducer according to one of claims 1 to 5, wherein the second metal body is provided with, on a base-end surface thereof, a plurality of heat-radiation fins that extend in the longitudinal-axis direction.
